(19) Europäisches Patentamt

European Patent Office

Office européen des brevets

(11) **EP 1 002 785 B1**

(12) **EUROPEAN PATENT SPECIFICATION**

(45) Date of publication and mention
of the grant of the patent:
**07.05.2003 Bulletin 2003/19**

(51) Int Cl.[7]: **C07C 51/12**, C07C 53/08

(21) Application number: **99308792.3**

(22) Date of filing: **04.11.1999**

(54) **Process for the production of acetic acid**

Verfahren zur Herstellung von Essigsäure

Procédé pour la préparation de l'acide acétique

(84) Designated Contracting States:
**DE FR GB**

(30) Priority: **19.11.1998 GB 9825424**

(43) Date of publication of application:
**24.05.2000 Bulletin 2000/21**

(73) Proprietor: **BP Chemicals Limited**
**London EC2M 7BA (GB)**

(72) Inventors:
• **Watson, Derrick John**
**Salt End, Hull, HU12 8DS (GB)**

• **Muskett, Michael James**
**Salt End, Hull, HU12 8DS (GB)**

(74) Representative: **Perkins, Nicholas David et al**
**BP International Limited**
**Patents and Agreements Division,**
**Chertsey Road**
**Sunbury-on-Thames, Middlesex TW16 7LN (GB)**

(56) References cited:
**EP-A- 0 752 406**       **US-A- 4 111 982**
**US-A- 5 352 415**       **US-A- 5 374 774**

## Description

The present invention relates in general to a process for the production of acetic acid and in particular to a process for the production of acetic acid by the carbonylation of methanol and/or a reactive derivative thereof in the presence of a Group VIII noble metal catalyst and a hydrocarbyl halide co-catalyst.

The production of acetic acid by the carbonylation of methanol and/or a reactive derivative thereof in the presence of a Group VIII noble metal catalyst and a hydrocarbyl halide co-catalyst, optionally in the presence of one or more promoters is well-known. Thus, processes using rhodium as the Group VIII noble metal catalyst are known from, for example, GB-A-1,233,121; EP-A-0384652; and EP-A-0391680. Processes using iridium as the Group VIII noble metal catalyst are known from, for example, GB-A-1234121; US-A-3772380; DE-A-1767150; EP-A-0616997; EP-A-0618184; EP-A-0618183; and EP-A-0657386. Processes using either rhodium or iridium as the Group VIII noble metals are operated on a commercial scale throughout the world.

EP-A-0 752 406, US-A-5 352 415 and US-A-5 374 774 relate to the production of acetic acid by carbonylation of methanol.

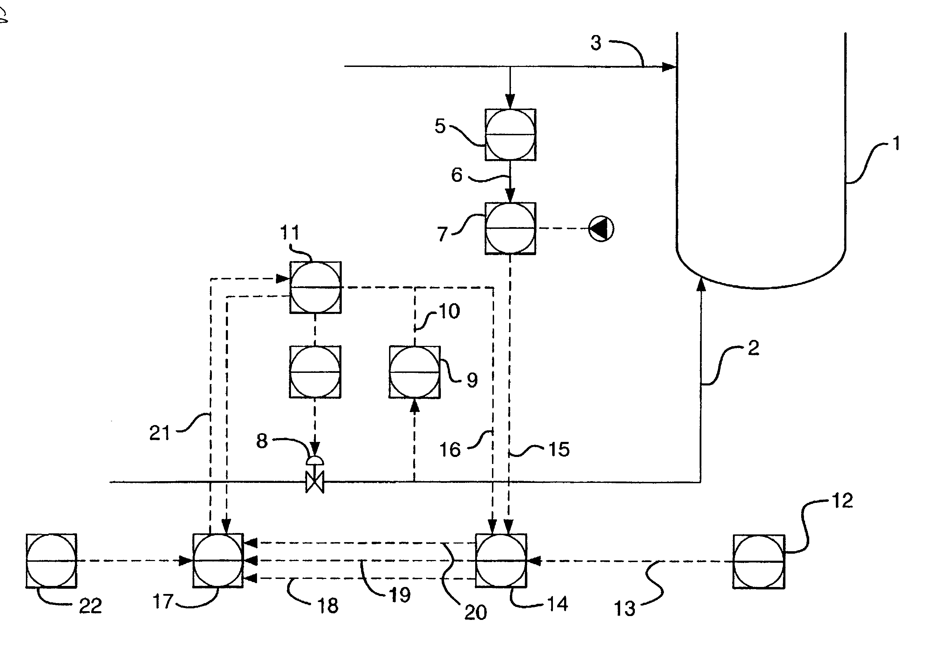

Howard et al in Catalysis Today, 18 (1993) 325-354 describe rhodium and iridium catalysed carbonylation of methanol to acetic acid. The continuous rhodium-catalysed, homogeneous methanol carbonylation process is said to consist of three basic sections; reaction, purification and off-gas treatment. The reaction section comprises a reactor, operated at elevated temperature and pressure, and a flash vessel. Methanol and gaseous carbon monoxide are fed to the reactor wherein there is maintained a liquid reaction composition comprising methyl acetate, water, rhodium or iridium catalyst, methyl iodide co-catalyst, optionally at least one promoter, and comprising the remainder of the composition acetic acid. Liquid reaction composition is withdrawn from the reactor and is passed through a flashing valve to the flash tank where the majority of the lighter components of the liquid reaction composition (methyl iodide, methyl acetate and water) together with product acetic acid are vaporised. The vapour fraction is then passed to the purification section whilst the liquid fraction (comprising the rhodium catalyst in acetic acid) is recycled to the reactor (see Figure 2 of Howard et al). The purification section is said to comprise a first distillation column (the light ends column), a second distillation column (the drying column) and a third distillation column (the heavy ends column) (see Figure 3 of Howard et al). In the light ends column methyl iodide and methyl acetate are removed overhead along with some water and acetic acid. The vapour is condensed and allowed to separate into two phases in a decanter, both phases being returned to the reactor. Wet acetic acid is removed from the light ends column as a side draw and is fed to the drying column where water is removed overhead and an essentially dry acetic acid stream is removed from the base of the distillation zone. From Figure 3 of Howard et al it can be seen that the overhead water stream from the drying column is recycled to the reaction section. Heavy liquid by-products are removed from the base of the heavy ends column with product acetic acid being taken as a side stream.

Not all the carbon monoxide which is fed to the reactor is carbonylated, the excess being vented from the reactor as high pressure vent and from the purification section as low pressure vent. These vents are combined after being scrubbed for removal of, for example methyl acetate and methyl iodide, which are returned to the reactor, and the carbon monoxide content of the combined vent gas is measured. The carbon monoxide mass flow out of the unit is then calculated. A simple subtraction of this number from the total carbon monoxide fed gives a good indication of how much carbon monoxide is being used in the carbonylation.

It has been found that in plant upsets where activity in the reactor has dropped off either quickly or over longer periods of time that the resulting imbalance in molar feed ratio causes operational problems due to increasing amounts of methyl acetate being created. This arises because, as the activity drops, the methanol feed remains at the same rate but the methyl acetate it creates on contact with acetic acid is not then consumed quickly enough by carbon monoxide due to the drop in activity. Thus, the methyl acetate builds in the reactor and consequently also in the decanter and thereby causes operational problems which can necessitate large feed rate cuts in order to recover control in the reactor. Such production upsets can be expensive in terms of lost production. Another circumstance which can lead to operational problems is that in which there is a carbon monoxide feed cut or loss due to supply problems. In this event there is a consequential methyl acetate build up due to the continued supply of methanol at the same rate.

The problem sought to be solved by the present invention is that of controlling the methyl acetate concentration in the liquid reaction composition in the reactor and thereby avoiding the operational problems which can arise from build-up thereof in the reactor as described hereinabove. It has been found that the problem can be solved by monitoring the ratio of methanol and/or reactive derivative thereof to carbon monoxide being converted to acetic acid and controlling the feed rate of the methanol and/or reactive derivative thereof in response thereto.

Accordingly, the present invention provides a process for the continuous production of acetic acid by feeding methanol and/or a reactive derivative thereof and carbon monoxide to a carbonylation reactor wherein there is maintained a liquid reaction composition comprising methyl acetate, water, Group VIII noble metal carbonylation catalyst, hydrocarbyl halide co-catalyst, optionally at least one promoter, and acetic acid, wherein the methyl acetate concentration in the liquid reaction composition is maintained at a pre-determined value by monitoring the ratio of methanol and/or reactive derivative thereof to carbon monoxide being converted to acetic acid and adjusting the feed rate of the methanol and/or reactive derivative thereof in response thereto in a manner such that the methyl acetate concentration is maintained at the pre-determined value.

The ratio of methanol and/or reactive derivative thereof to carbon monoxide being converted to acetic acid, hereinafter referred to as the Ratio Controller Process Value (R), is defined by the following relationship:-

$$R = M/(C - O) \qquad (I)$$

wherein M = Methanol and/or reactive derivative feed flow (molar),

C = Carbon monoxide feed flow (molar), and

O = Carbon monoxide in combined off-gas flow (molar).

Under normal steady state running conditions the value of R should be substantially equal to unity. Under conditions whereby there is a loss of activity in the reactor, by for example a small fall in temperature or a change in water content, the value of R will increase above unity. The extent of this increase will determine the nature of the responsive action by way of adjustment of the methanol feed rate to be taken.

The description which follows will be for convenience confined to methanol as the feedstock, though it applies equally well to a methanol derivative.

The methanol feed flow is controlled in response to the value of R, as calculated by a Methanol Ratio Controller, suitably by a Methanol Flow Controller, which functions in a manner such that (i) when R is less than a pre-determined value (X) the Methanol Ratio Controller does nothing other than supervise and set a non-restrictive set point limit on the Methanol Flow Controller Value, (ii) when R is equal to or greater than the pre-determined value (X) a computer calculation is effected to determine what M would require to be to restore the value of R to unity, which value is written by the Ratio Controller to the set point high limit on the Methanol Flow Controller, which in turn responds by reducing the Methanol Feed Flow until the value of R equals unity, and (iii) when R is equal to unity the Ratio Controller resumes its supervision until the next time R exceeds the pre-determined value whereupon the aforesaid cycle is repeated.

The pre-determined value (X) of the Ratio Controller Process Value (R) may be any value desired, suitably a value in the range from 1.05 to 1.35, preferably from 1.10 to 1.25, more preferably from 1.10 to 1.20, for example 1.15.

The Methanol Ratio Controller, it may be noted, will never function to increase the Methanol Feed Flow; it will only ever cut the Flow in the event of activity loss in the reactor. During initial plant start-up, when methanol is required to be fed quickly to the reactor, the Controller should be rendered inoperative. When the Controller is inoperative a maximum set point high limit is desirably written into the Methanol Flow Controller automatically so as not to inhibit methanol feed rates.

Although the relationship (I) is defined in molar terms, it is not absolutely necessary to do so. The various parameters may be expressed in terms other than molar if desired, for example volume or mass terms. It will be evident to the person skilled in the art that the threshold value of X will depend upon the basis for the definition of the ratio, R.

The carbon monoxide feed flow, carbon monoxide flow in the off-gas and methanol feed flow are measured and used in determining the ratio R. Preferably, the carbon monoxide feed flow is measured and used as a 10 minute rolling average.

There is fed to the carbonylation reactor methanol and/or reactive derivative thereof. Suitable reactive derivatives of methanol include methyl acetate, dimethyl ether and methyl halides, of which methyl iodide is preferred.

Also fed to the carbonylation reactor is carbon monoxide. The carbon monoxide reactant may be essentially pure or may contain inert impurities such as carbon dioxide, methane, nitrogen, noble gases, water and $C_1$ to $C_4$ paraffinic hydrocarbons. Hydrogen may be present or absent, preferably absent. The partial pressure of carbon monoxide may suitably be in the range from 1 to 70 barg, preferably from 1 to 35 barg, and more preferably 1 to 15 barg.

There is maintained in the carbonylation reactor a liquid reaction composition comprising methyl acetate, water, Group VIII noble metal carbonylation catalyst, hydrocarbyl halide co-catalyst, optionally at least one promoter, and acetic acid.

Methyl acetate, besides being optionally fed to the carbonylation reactor, is formed by esterification. It may suitably be present in the liquid reaction composition in an amount from 1 to 70% by weight, preferably from 1 to 35% by weight, more preferably 1 to 20% by weight.

Water may be formed in situ in the carbonylation reaction, for example by the esterification reaction between alcohol reactant and acetic acid product. Water may be introduced to the carbonylation reactor together with or separately from the other liquid reactants such as esters, for example methyl acetate. Water may be separated from reaction composition withdrawn from the reactor and recycled in controlled amounts to maintain the required concentration in the carbonylation reaction composition. The concentration of water in the liquid carbonylation reaction composition may be at least 0.1% by weight. Typically, and depending upon the other components of the liquid reaction composition, the water concentration in the liquid carbonylation reaction composition may be at least 0.1% by weight. Typically, and depending upon the other components of the liquid reaction composition, the water concentration in the liquid carbonylation reaction composition may be at least 0.1% by weight and up to 30% by weight, preferably up to 15% by weight, most preferably the water concentration is 2 to 8% by weight.

As regards the carbonylation catalyst, of the Group VIII noble metals rhodium and iridium are preferred. The process of the invention is particularly applicable to the use of an iridium catalyst because of the faster carbonylation rates generally achievable thereby and the correspondingly enhanced methyl acetate accumulation accompanying an activity decrease.

Where the catalyst is a rhodium carbonylation catalyst, the reaction composition may contain any rhodium-containing compound which is soluble in the liquid reaction composition. It may be added to the liquid carbonylation reaction composition for the carbonylation reaction in any suitable form which dissolves in the liquid reaction composition or is convertible to a soluble form. Examples of suitable rhodium-containing compounds which may be added to the liquid reaction composition include $[Rh(CO)_2Cl]_2$, $[Rh(CO)_2I]_2$, $[Rh(Cod)Cl]_2$, rhodium (III) chloride, rhodium (III) chloride trihydrate, rhodium (III) bromide, rhodium (III) iodide, rhodium (III) acetate, rhodium dicarbonylacetylacetone, $RhCl_3 (PPh_3)_3$ and $RhCl(CO)(PPh_3)_2$.

Where the catalyst composition is an iridium carbonylation catalyst, again the iridium compound may be added to the liquid carbonylation reaction composition for the carbonylation reaction in any suitable form which dissolves in the liquid reaction composition or is convertible to a soluble form.

Preferably the iridium may be used as a chloride free compound such as acetates which are soluble in one or more of the liquid reaction components, e.g. water and/or acetic acid and so may be added to the reaction as solutions therein. Examples of such iridium-containing compounds which may be added to the liquid reaction composition include $IrCl_3$, $IrI_3$, $IrBr_3$, $[Ir(CO)_2I]_2$, $[Ir(CO)_2Cl]_2$, $[Ir(CO)_2Br]_2$, $[Ir(CO)_4I_2]^-H^+$, $[Ir(CO)_2Br_2]^-H^+$, $[Ir(CO)_2I_2]^-H^+$, $[Ir(CH_3)I_3(CO)_2]^-H^+$, $Ir_4(CO)_{12}$, $IrCl_3.4H_2O$, $IrBr_3.4H_2O$, $Ir_3(CO)_{12}$, iridium metal, $Ir_2O_3$, $IrO_2$, $Ir(acac)(CO)_2$, $Ir(acac)_3$, iridium acetate, $[Ir_3O(OAC)_6(H_2O)_3][OAc]$ and hexachloroiridic acid $H_2[IrCl_3]$, preferably chloride free complexes of iridium such as acetates, oxalates and acetoacetates.

Preferably, the concentration of the catalyst in the liquid reaction composition is in the range of from 50 to 5000 ppm by weight of metal, preferably 100 to 2500 ppm by weight of metal.

Where the catalyst composition comprises iridium, the composition may optionally comprise a metallic promoter. The metallic promoter may suitably be one or more of osmium, rhenium, ruthenium, cadmium, mercury, zinc, gallium, indium and tungsten. Preferably, the promoter is selected from ruthenium and osmium and most preferably is ruthenium. The promoter may comprise any promoter metal-containing compound which is soluble in the liquid reaction compositions. The promoter may be added to the liquid reaction composition in any suitable form which dissolves in the liquid reaction composition or is convertible to soluble form. Preferably, the promoter may be used as chloride free compounds such as acetates which are soluble in one or more of the liquid reaction composition components e.g. water and/or acetic acid and so may be added to the reaction as a solution therein.

Examples of suitable ruthenium-containing compounds which may be used as promoter include ruthenium (III) chloride, ruthenium (III) chloride trihydrate, ruthenium (IV) chloride, ruthenium (III) bromide, ruthenium (III) iodide, ruthenium metal, ruthenium oxides, ruthenium (III) formate, $[Ru(CO)_3I_3]^-H^+$, tetra(aceto)chlororuthenium(II, III), ruthenium (III) acetate, ruthenium (III) propionate, ruthenium (III) butyrate, ruthenium pentacarbonyl, trirutheniumdodecacarbonyl and mixed ruthenium halocarbonyls such as dichlorotricarbonylruthenium (II) dimer, dibromotricarbonylruthenium (II) dimer, and other organoruthenium complexes such as tetrachlorobis(4-cymene)diruthenium(II), tetrachlorobis (benzene)diruthenium(II), dichloro(cycloocta-1,5-diene)ruthenium (II) polymer and tris(acetylacetonate)ruthenium (III).

Examples of suitable osmium-containing compounds which may be used as sources of co-promoter include osmium (III) chloride hydrate and anhydrous, osmium metal, osmium tetraoxide, triosmiumdodecacarbonyl, pentachloro-μ-nitrododiosmium and mixed osmium halocarbonyls such as tricarbonyldichloroosmium (II) dimer and other organoosmium complexes.

Examples of suitable rhenium-containing compounds which may be used as sources of co-promoter include $Re_2(CO)_{10}$, $Re(CO)_5Cl$, $Re(CO)_5Br$, $Re(CO)_5I$, $ReCL_3.xH_2O$ $ReCl_5.yH_2O$ and $[{ReCO)_4I}_2]$.

Examples of suitable cadmium-containing compounds which may be used as sources of co-promoter include $Cd(OAc)_2$, $CdI_2$, $CdBr_2$, $CdCl_2$, $Cd(OH)_2$, and cadmium acetylacetonate.

Examples of suitable mercury-containing compounds which may be used as sources of co-promoter include $Hg(OAc)_2$, $HgI_2$, $HgBr_2$, $HgCl_2$, $Hg_2I_2$, and $Hg_2Cl_2$.

Examples of suitable zinc-containing compounds which may be used as sources of co-promoter include $Zn(OAc)_2$, $Zn(OH)_2$, $ZnI_2$, $ZnBr_2$, $ZnCl_2$, and zinc acetylacetonate.

Examples of suitable gallium-containing compounds which may be used as sources of co-promoter include gallium acetylacetonate, gallium acetate, $GaCl_3$, $GaBr_3$, $GaI_3$, $Ga_2Cl_4$ and $Ga(OH)_3$.

Examples of suitable indium-containing compounds which may be used as sources of co-promoter include indium acetylacetonate, indium acetate, $InCl_3$,

$InBr_3$, $InI_3$, $InI$ and $In(OH)_3$.

Examples of suitable tungsten-containing compounds which may be used as sources of co-promoter include $W(CO)_6$, $WCl_4$, $WCl_6$, $WBr_5$, $WI_2$, or $C_9H1_2 W(CO)_3$ and any tungsten chloro-, bromo- or iodo-carbonyl compound.

Preferably, the promoter-containing compounds are free of impurities which provide or generate in-situ ionic iodides which may inhibit the reaction, for example, alkali or alkaline earth metal or other metal salts.

Preferably, the promoter is present in an effective amount up to the limit of its solubility in the liquid reaction compositions and/or any liquid process streams recycled to the carbonylation reaction zones from the acetic acid recovery stage. The promoter is suitably present in the liquid reaction compositions at a molar ratio of each promoter (when present): iridium in the range 0.1 : 1 to 100 :1, preferably in the range 1 : to 10 : 1. The beneficial effect of a promoter such as ruthenium has been found to be greatest at the water concentration which gives the maximum carbonylation rate at any defined methyl acetate and methyl iodide concentration. A suitable promoter concentration is 400 to 7000 ppm.

Using rhodium as the carbonylation catalyst the use of iodide promoters is preferred. Both inorganic and organic iodides may be employed. Suitable inorganic iodides include alkali metal and alkaline earth metal iodides. A preferred metal iodide is lithium iodide. The iodides may be added as such or in the form of salts, for example carboxylate salts, such as acetates, which are convertible to iodides under the carbonylation conditions. Alternatively, organic iodides, suitably selected from quaternary ammonium, pyridinium and picolinium iodides may be employed.

There is employed as co-catalyst in the liquid reaction composition a hydrocarbyl halide. The hydrocarbyl halide may be an iodide or a bromide and is preferably an iodide. Preferably the co-catalyst is an alkyl iodide, more preferably methyl iodide. A suitable co-catalyst concentration in the liquid reaction composition is in the range from 1 to 30% by weight, more preferably 1 to 20% by weight.

The total pressure of the carbonylation process is suitably in the range 10 to 100 barg. The temperature at which the carbonylation process is operated is suitably in the range from 100 to 300°C, preferably in the range from 150 to 220°C.

An advantage of the process of the present invention is that minor upsets on the plant, such as a small temperature deviation, do not lead to larger compositional problems.

The invention will now be described by way of example only and with reference to the drawing which shows a schematic representation of the methanol and carbon monoxide feed flow monitors and controllers to a liquid phase carbonylation reactor. The description which follows will be for convenience confined to methanol as the feedstock, though it applies equally well to a methanol derivative.

Referring to the drawing, a carbonylation reactor (1) is provided with an inlet for methanol reactant (2) and an inlet for carbon monoxide reactant (3). The carbon monoxide inlet is provided with a flow rate monitor (5) connected through signal line (6) to a flow indicator controller (7). The methanol inlet (2) is provided with a supply of methanol through flow control valve (8) and has a flow rate monitor (9) connected through signal line (10) to a flow indicator controller (11).

A flow rate monitor (12) is provided to measure the flow of off-gas from the carbonylation reactor after it has passed through absorbers (not shown) to remove organic components. The off-gas flow rate monitor (12) is connected through signal line (13) to a flow rate calculator (14). This calculator is also provided with a signal line (15) to receive signals from the carbon monoxide feed rate controller (7) and with a signal line (16) to receive signals from the methanol feed rate monitor (9).

Logic controller (17) is provided with signal lines (18), (19) and (20) from the calculator (14).

In use, the methanol feed flow is measured by monitor (9) passed through signal line (16) to the calculator (14) which calculates the molar flow rate. The carbon monoxide feed and off-gas flows are measured by monitors (5) and (12) respectively and the flow rates passed through signal lines (15) and (13) respectively to calculator (14) which calculates the molar carbon monoxide consumption. Calculator (14) then calculates the ratio R of methanol feed flow divided by carbon monoxide consumed and sends the result along signal line (20) to logical controller (17). The calculator (14) also calculates the required methanol feed flow which would be required to give ratios R of 1 and 1.15 and send these signals to logic controller (17) down signal lines (19) and (18) respectively.

If the ratio R is greater than a predetermined value X, for example 1.15, the logic controller (17) sends a signal along signal line (21) to the methanol flow indicator controller (11) to set the setpoint high limit to a value required to reduce the ratio R to 1, received from calculator (14) along line (19). These signals are maintained until the calculated value of R falls to 1 or less when the logical controller sends a signal to the methanol flow indicator controller to set the setpoint high limit to a value which would give an R of 1.15, received from calculator (14) along signal line (20). In this way, the flow of methanol to the carbonylation reactor is limited in conditions when the reactivity of the system is reduced.

The logic controller is also provided with means (22) for manually overriding the system if required.

## Claims

1. A process for the continuous production of acetic acid by feeding methanol and/or a reactive derivative thereof and carbon monoxide to a carbonylation reactor wherein there is maintained a liquid reaction composition comprising methyl acetate, water, Group VIII noble metal carbonylation catalyst, hydrocarbyl halide co-catalyst, optionally at least one promoter, and acetic acid, wherein the methyl acetate concentration in the liquid reaction composition is maintained at a pre-determined value by monitoring the ratio of methanol and/or reactive derivative thereof to carbon monoxide being converted to acetic acid and adjusting the feed rate of the methanol and/or reactive derivative thereof in response thereto in a manner such that the methyl acetate concentration is maintained at the pre-determined value.

2. A process as claimed in claim 1 in which the methanol feed flow is controlled in response to a value of R, as calculated by a Methanol Ratio Controller, wherein

$$R = M/(C-O) \qquad (I)$$

wherein

M = Methanol and/or reactive derivative feed flow (molar),
C = Carbon monoxide feed flow (molar), and
O = Carbon monoxide in combined off-gas flow (molar).

3. A process as claimed in claim 2 in which the Methanol Flow Controller, functions in a manner such that when R is equal to or greater than a pre-determined value (X) a computer calculation is effected to determine what M would require to be, to restore the value of R to unity, which value is written by the Ratio Controller to the set point high limit on the Methanol Flow Controller, which in turn responds by reducing the Methanol Feed Flow until the value of R equals unity.

4. A process as claimed in claim 3 in which when R is less than the pre-determined value (X) the Methanol Ratio Controller sets a non-restrictive set point limit on the Methanol Flow Controller Value.

5. A process as claimed in claim 3 or claim 4 in which X is in the range from 1.05 to 1.35.

6. A process as claimed in claim 5 in which X is in the range 1.10 to 1.25.

7. A process as claimed in claim 6 in which X is in the range 1.10 to 1.20.

8. A process as claimed in any one of claims 3 to 6 in which during start-up, when methanol is required to be fed quickly to the reactor, the Controller is rendered inoperative and a maximum set point high limit is written into the Methanol Flow Controller automatically so as not to inhibit methanol feed rate.

9. A process as claimed in any one of the preceding claims in which the Group VIII noble metal carbonylation catalyst is rhodium or iridium.

10. A process as claimed in claim 9 in which the Group VIII noble catalyst is iridium and the optional promoter is elected from the group consisting of ruthenium, osmium, rhenium, cadmium, mercury, zinc, gallium, indium, tungsten and mixtures thereof.

11. A process as claimed in any one of the preceding claims in which methyl acetate is present in the liquid reaction composition in an amount from 1 to 70 % by weight.

## Patentansprüche

1. Verfahren zur kontinuierlichen Herstellung von Essigsäure durch Zuführen von Methanol und/oder einem reaktiven Derivat davon und Kohlenmonoxid zu einem Carbonylierungsreaktor, worin eine flüssige Reaktionszusammensetzung, die Essigsäuremethylester, Wasser, Edelmetallcarbonylierungskatalysator der Gruppe VIII, Kohlenwasserstoffhalogenid-Cokatalysator, gegebenenfalls mindestens einen Promotor und Essigsäure umfasst, gehalten wird, wobei die Essigsäuremethylesterkonzentration in der flüssigen Reaktionszusammensetzung bei einem vorbestimmten Wert gehalten wird, durch Verfolgen des Verhältnisses von Methanol und/oder reaktivem Derivat davon zu Kohlenmonoxid, welche zu Essigsäure umgewandelt werden, und Einstellen der Zuführungsrate von Methanol und/oder reaktivem Derivat davon in Reaktion dazu in einer derartigen Weise, dass die Essigsäuremethylesterkonzentration bei dem vorbestimmten Wert gehalten wird.

2. Verfahren nach Anspruch 1, wobei der Methanolzuführungsstrom in Reaktion auf einen Wert von R, wie berechnet durch einen Methanol Ratio Controller, gesteuert wird, worin

$$R = M/(C-O) \qquad (I),$$

worin

M = Methanol und/oder Zuführungsstrom von reaktivem Derivat (molar),

C = Kohlenmonoxidzuführungsstrom (molar) und

O = Kohlenmonoxid in vereinigtem Abgasstrom (molar).

3. Verfahren nach Anspruch 2, wobei der Methanol Flow Controller in einer derartigen Weise wirkt, dass wenn R gleich oder größer als der vorbestimmte Wert (X) ist, eine Computerberechnung bewirkt wird, um zu bestimmen, was M sein sollte, um den Wert von R auf Eins zurückzustellen (restore unity), welcher Wert durch den Ratio Controller zu dem Sollwert-oberer Grenzwert auf dem Methanol Flow Controller gespeichert wird, welcher wiederum durch Vermindern des Methanol Feed Flow reagiert bis der Wert von R Eins (unity) entspricht.

4. Verfahren nach Anspruch 3, wobei wenn R weniger als der vorbestimmte Wert (X) ist, der Methanol Ratio Controller einen nicht begrenzenden Sollwert-Grenzwert auf dem Methanol-Flow Controller-Value einstellt.

5. Verfahren nach Anspruch 3 oder Anspruch 4, wobei X im Bereich von 1,05 bis 1,35 liegt.

6. Verfahren nach Anspruch 5, wobei X im Bereich von 1,10 bis 1,25 liegt.

7. Verfahren nach Anspruch 6, wobei X im Bereich von 1,10 bis 1,20 liegt.

8. Verfahren nach einem der Ansprüche 3 bis 6, wobei während des Anfahrens, wenn Methanol sehr schnell dem Reaktor zugeführt werden muss, der Controller wirkungslos gemacht wird und ein maximaler Sollwert-Grenzwert automatisch in den Methanol Flow Controller eingespeichert wird, sodass die Methanolzuführungsrate nicht gehemmt wird.

9. Verfahren nach einem der vorangehenden Ansprüche, wobei der Edelmetallcarbonylierungskatalysator der Gruppe VIII Rhodium oder Iridium ist.

10. Verfahren nach Anspruch 9, wobei der Edelmetallkatalysator der Gruppe VIII Iridium ist und der wahlweise Promotor aus der Gruppe, bestehend aus Ruthenium, Osmium, Rhenium, Cadmium, Quecksilber, Zink, Gallium, Indium, Wolfram und Gemischen davon, ausgewählt ist.

11. Verfahren nach einem der vorangehenden Ansprüche, wobei Essigsäuremethylester in der flüssigen Reaktionszusammensetzung in einer Menge von 1 bis 70 Gewichtsprozent vorliegt.

**Revendications**

1. Procédé pour la production continue d'acide acétique en introduisant du méthanol et/ou un de ses dérivés réactifs et du monoxyde de carbone dans un réacteur de carbonylation dans lequel est maintenue une composition réactionnelle liquide comprenant de l'acétate de méthyle, de l'eau, un catalyseur de carbonylation à base d'un métal noble du groupe VIII, un cocatalyseur consistant en un halogénure d'hydrocarbyle, facultativement au moins un promoteur et de l'acide acétique, la concentration d'acétate de méthyle dans la composition réactionnelle liquide étant maintenue à une valeur prédéterminée en contrôlant le rapport du méthanol et/ou de son dérivé réactif au monoxyde de carbone convertis en acide acétique et en ajustant la vitesse d'introduction du méthanol et/ou de son dérivé réactif en réponse à ce contrôle de telle sorte que la concentration en acétate de méthyle soit maintenue à la valeur prédéterminée.

2. Procédé suivant la revendication 1, dans lequel le débit d'introduction du méthanol est régulé en réponse à une valeur de R, de la manière calculée par un système de commande des proportions de méthanol, où

$$R = M / (C - O) \qquad (I)$$

dans laquelle

M = débit (molaire) d'introduction du méthanol et/ou de son dérivé réactif,

C = débit d'introduction (molaire) de monoxyde de carbone, et

O = monoxyde de carbone dans le courant (molaire) des gaz résiduaires réunis.

3. Procédé suivant la revendication 2, dans lequel le système de commande de débit de méthanol fonctionne de telle sorte que, lorsque R est égal ou supérieur à une valeur prédéterminée (X), un calcul informatique est effectué pour déterminer quelle valeur de M serait requise, pour rétablir la valeur de R à l'unité, valeur qui est écrite par le système de commande de rapport à la limite supérieure de point de réglage sur le système de commande de débit de méthanol, qui répond alors en réduisant le débit d'introduction de méthanol jusqu'à ce que la valeur de R soit égale à l'unité.

4. Procédé suivant la revendication 3, dans lequel, lorsque R est inférieur à la valeur prédéterminée (X), le système de commande des proportions de méthanol fixe une limite de point de réglage non restrictive à la valeur du système de commande de débit de méthanol.

5. Procédé suivant la revendication 3 ou la revendication 4, dans lequel X est compris dans l'intervalle de 1,05 à 1,35.

6. Procédé suivant la revendication 5, dans lequel X est compris dans l'intervalle de 1,10 à 1,25.

7. Procédé suivant la revendication 6, dans lequel X est compris dans l'intervalle de 1,10 à 1,20.

8. Procédé suivant l'une quelconque des revendications 3 à 6, dans lequel, au cours du démarrage, lorsque l'introduction rapide de méthanol dans le réacteur est requise, le système de commande est rendu inopérant et une limite supérieure de point de réglage maximale est écrite automatiquement dans le système de commande de débit de méthanol de manière à ne pas inhiber la vitesse d'introduction de méthanol.

9. Procédé suivant l'une quelconque des revendications précédentes, dans lequel le catalyseur de carbonylation à base d'un métal noble du groupe VIII est le rhodium ou l'iridium.

10. Procédé suivant la revendication 9, dans lequel le catalyseur à base d'un métal noble du groupe VIII est l'iridium et le promoteur facultatif est choisi dans le groupe consistant en ruthénium, osmium, rhénium, cadmium, mercure, zinc, gallium, indium, tungstène et leurs mélanges.

11. Procédé suivant l'une quelconque des revendications précédentes, dans lequel de l'acétate de méthyle est présent dans la composition réactionnelle liquide en une quantité de 1 à 70 % en poids.

EP 1 002 785 B1